# EUROPEAN PATENT SPECIFICATION

(11) **EP 1 504 046 B1**
(45) Date of publication and mention of the grant of the patent: **12.07.2006**
(21) Application number: 03808379.6
(22) Date of filing: 15.05.2003
(51) Int. Cl.: C08G 63/00, C08G 63/06, A61K 47/48, A61M 31/00, C08G 69/44

(54) **TRI-BLOCK POLYMERS FOR NANOSPHERE-BASED DRUG OR GENE DELIVERY**
TRIBLOCKPOLYMERE FÜR ARZNEISTOFF- ODER GENZUFUHR AUF NANOKUGELBASIS
POLYMERES TRIBLOCS POUR ADMINISTRATION DE GENE OU DE MEDICAMENT A BASE DE NANOSPHERES

(30) Priority: 15.05.2002 US 378042 P
(43) Date of publication of application: 09.02.2005
(73) Proprietor: Rutgers, The State University, New Brunswick, New Jersey 08903 (US)
(72) Inventor: KOHN, Joachim, South Plainfield, NJ 07080 (US); VEBERT-NARDIN, Corinne, CH-4142 Muenchenstein (CH); BOLIKAL, Durgadas, Piscataway, NJ 08854 (US); SEYDA, Agnieszka, Piscataway, NJ 08854 (US)
(74) Representative: Bergstrand, Mikael Gudmundsson
(86) International application number: PCT/US2003/015600
(87) International publication number: WO 2004/039944

(56) References cited:
- US-A- 5 587 507
- US-A- 6 103 255
- US-A1- 2001 046 505
- US-B1- 6 359 102

## Description

### BACKGROUND OF THE INVENTION

A promising way for the treatment of both acquired and genetic diseases lies in gene therapy, i.e., the transfection of a suitable genetic material into target cells via a vector which characteristics are currently well defined. For instance, to allow both intravascular administration and uptake by the cells, the size of the carrier is limited to about 150 nm. Two uptake mechanisms might take place: Fusion of the vector with the cell membrane that permits a direct release of the material in the intracellular space is possible but less probable than phagocytosis of the vector by the cell. In the latter case, the vector has to escape the endosome and release the material into the intracellular medium before being trapped and degraded within a lysosome. However, in the two cases, the vector has to be non-cytotoxic and biodegradable while affording a protection of the genetic material all the way along the transfection pathway.

Whereas the vector characteristics are well-defined, gene therapy is still limited by the absence of efficient and harmless vectors. Viral vectors are efficient and able to target a wide range of cells. However, they may suffer from both the drawbacks of immunogenecity and potential mutagenicity. The same problems hold for proteoliposomes used for the protein-mediated encapsulation of a genetically engineered viral genome. Synthetic, non-viral vectors offer an attractive alternative to viral vectors but suffer generally from being far less efficient in their ability to transfect cells than viral vectors.

Currently, a highly interesting challenge in the biomaterials field is the preparation of suitable carriers for drug or genetic material. Because of their ability to protect the encapsulated material against enzymatic degradation for example, capsules appear to be the more suitable vehicles. Nanocapsules, i.e., carriers with a size in the subnanometer range are desirable for intravascular administration. For this purpose, the recent advances in supra-molecular chemistry allow designing materials of superior characteristics.

Given the above-stated difficulties, encapsulation appears to be the most interesting technique of gene carrier preparation. Currently, liposomes are used to entrap nucleic acids, but electrostatic interactions still occur between the lipids and the cell membrane or the DNA, which limit the transfection efficiency. Additionally, the poor stability of liposomes over time and their immunogenicity lead to their rapid clearance from the blood stream.

Of particular interest is the self-assembly of block copolymers. Similar to low molecular weight lipid or surfactant molecules, amphiphilic block copolymers consist of at least two parts, a water friendly portion and a hydrophobic block. Those amphiphilic block copolymers, driven by their hydrophobicity, self-assemble in aqueous solution. At high concentrations, they build lamellar liquid crystalline phases whereas, in dilute aqueous solution, they form superstructures of various shapes like micelles or vesicular structures.

Those block copolymer molecules have to be regarded as the analogues from lipids or surfactants. However, due to their slower dynamics and higher molecular weight, their self-assembly has been shown to lead to much more stable superstructures. Furthermore, liposomes, e.g., spherically closed lipid bilayers, are rapidly recognized by the immune system and cleared from the blood stream. Due to the wide variety of block copolymer chemistry one can prepare an entirely synthetic material to avoid any immunogenic reaction.

A suitable neutral amphiphilic block copolymer forms spontaneously nanometer-sized, well-defined hollow sphere structures in dilute aqueous solution. These structures can be viewed as the high molecular weight analogues of lipid or surfactant molecules. However due to their slow dynamic, they form much more stable superstructures than conventional liposomes.

Although it is well known that suitable block copolymers can form nanocapsules, few were designed to self-assemble into hollow sphere structures in dilute aqueous solution. Only one example of spontaneous aggregation of an amphiphilic block copolymer has been reported with a poly(methyloxazoline)-block-poly(dimethyl-siloxane)-block-poly(methyloxazoline), PMOXA-PDMS-PMOXA triblock copolymer. Injection combined with extrusion techniques leads to the formation of vesicles whose size can be controlled between 50 and 500 nm. However, there remains a need for non-cytotoxic, biodegradable triblock copolymer vesicles.

### SUMMARY OF THE INVENTION

DNA complexes formed with amphiphilic ABA triblock copolymers built from non-cytotoxic and biodegradable blocks have been discovered that show an increased ability to spontaneously mediate cell transfection. It has now been determined that the resulting self-assembly is still non-cytotoxic and is also useful for controlled drug delivery.

Accordingly, a new family of triblock copolymers is disclosed that has an unprecedented and novel combination of properties. The copolymers are entirely prepared from non-toxic and biocompatible building blocks,

The triblock copolymers are derived from water-soluble, hydrophilic, and non-toxic polymer end blocks and a hydrophobic middle block polyarylate oligomer of a biocompatible, non-toxic aliphatic or aromatic diacid and a derivative of a tyrosine-derived diphenol. Thus, according to one aspect of the present invention, polyarylate triblock copolymers are provided having an A-B-A structure wherein each A end block is a water-soluble, hydrophilic, and non-toxic polymer end block; and
the B middle block is an polyarylate oligomer with the same or different repeating units having the structure of Formula I: 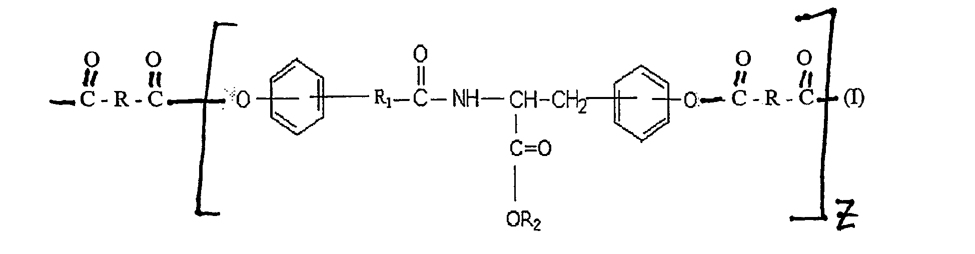 wherein Z is between 2 and to about 20, and preferably about 10, R₁ is CH=CH or (CH₂)ₙ
wherein n is from 0 to 18, inclusive; R₂ is selected from hydrogen and straight and branched alkyl and alkylaryl groups containing up to 18 carbon atoms; and R is selected from a bond or straight and branched alkyl and alkylaryl groups containing up to 18 carbon atoms. One or more of R, R₁ and R₂ may optionally contain an ether linkage.

The endblocks are preferably poly(alkylene oxides) having the structure of Formula II:

R₃-[(CH₂-)ₐCHR₃-O-]ₘ- (II)

wherein m for each A is independently selected to provide a molecular weight for each A between about 1000 and about 15,000 and R₃ for each A and within each A is independently selected from hydrogen and lower alkyl groups containing from one to four carbon atoms

The triblock copolymers self-assemble spontaneously to form hollow, biodegradable nanospheres with diameters ranging in diameter from about 5 to 200 nanometer with an unexpectedly low "critical aggregation concentration" (CAC) of 0.26 milimole/liter. Therefore, according to another aspect of the present invention, nanospheres of the triblock copolymers of the present invention are also provided, preferably in the size range of 5 to 200 nanometers (diameter). The low critical aggregation concentration (CAC) of only 0.26 millimole/liter means that the self-assembled polymer nanospheres remain stable even under very high dilution. Accordingly, these nanospheres are expected to be useful for the delivery of drugs and other actives even at very low concentration.

In particular, the polymer nanospheres of the present invention, by virtue of being hollow, can be used to encapsulate drugs, genetic materials (RNA, DNA, antisense oligonucleotides), or other active ingredients such as contrast agents, i.e., essentially any useful pharmaceutical or biological agent in the broadest sense, and provide a means for the prolonged release of the encapsulated materials. Therefore, according to yet another aspect of the present invention, nanospheres of the polymers of the present invention are provided in which an agent for administering to a patient in need thereof is encapsulated thereby. Yet another aspect of the invention provides compositions for delivering an active agent to a patient in need thereof containing a pharmaceutically acceptable carrier and an effective amount of nanospheres encapsulating the agent with the tribock copolymer of the present invention.

Preferred embodiments of this aspect of the invention provide nanosphere-encapsulated biologically or pharmaceutically active compounds, wherein the active compound nanospheres are present in an amount sufficient for effective site-specific or systemic delivery. The carrier may be an aqueous solution in which the nanospheres are suspended, or a polmeric drug delivery matrix. This aspect of the present invention includes embodiments in which the polymer nanospheres of the present invention function as a 'reservoir' for active agents within a polymeric matrix-based, controlled release device (such as a hydrogel or any of the other types of polymeric controlled release systems as described in P. Sinko and J. Kohn ("Polymeric drug delivery systems: An overview", in: *Polymeric Delivery Systems: Properties and Applications,* (M. A. El-Nokaly, D. M. Piatt and B. A. Charpentier, eds.), ACS Symposium Series, Vol. 520,**1993**, American Chemical Society, Washington, DC, 18-41.).

Given this utility of the polymer nanospeheres of the present invention, according to still yet another aspect of the present invention, methods are provided for site-specific or systemic delivery by administering to a patient in need thereof an effective amount of an active compound encapsulated by the polymer nanospheres of the present invention.

Plasmid vectors for cell transfection encapsulated by the nanospheres of the present invention are of a size suitable for gene delivery. Therefore, according to another aspect of the present invention, a composition for gene delivery is provided, which is a pharmaceutically acceptable solution or suspension of nanosphere-encapsulated plasmid vectors containing the gene to be delivered, wherein said nanospheres are formed from the triblock copolymer of the present invention. Another aspect of the present invention provides gene delivery methods, wherein a cell to be transfected is contacted with a gene delivery composition according to the present invention.

While certain of the above-mentioned properties are individually well-known in the prior art, the combination of properties within a single composition is new and represents a significant technological advance that has broad utility in the fields of drug and gene delivery and the controlled (or prolonged) release of active agents. Specifically, the family of triblock copolymers described herein has at least three major, distinguishing advantages over other triblock copolymers:
1. The family of triblock copolymers is fully resorbable after being introduced into a patient. As the compositions are derived exclusively of non-toxic building blocks, the triblock copolymers themselves as well as the expected degradation products in vivo are non-cytotoxic, and biocompatible.
2. The family of triblock copolymers self-assemble to form hollow nanospheres with the above-mentioned low critical aggregation concentration (CAC) and remain stable even under very high dilution.
3. The family of triblock copolymer provides a wide range of structural parameters which can be changed by those skilled in organic synthesis to derive triblock copolymers that are closely related to each other in overall chemical structure while allowing the tailoring of key properties (such as the rate of bioresorption, the physical characteristics of the nanospheres formed, and the release profiles obtained for encapsulated 'actives'.

A more complete appreciation of the invention and many other intended advantages can be readily obtained by reference to the following Detailed Description of the Preferred Embodiment and claims, which disclose the principles of the invention and the best modes which are presently contemplated for carrying them out.

### BRIEF DESCRIPTION OF THE DRAWINGS

FIG.1 is a freeze-fracture transmission electron micrograph of the nanosphere self-asembly of a PEG-oligo(DTO suberate)-PEG triblock copolymer of the present invention;
FIG.2 is a static light scattering Zimm plot of the nanospheres of FIG. 1 in dilute aqueous solution;
FIG.3 is a dynamic light scattering Zimm plot of the nanospheres of FIG. 1 in dilute aqueous solution;
FIG. 4 is a cytotransmission electron micrograph of the nanosphere of FIG. after self-assembly in dilute aqueous solution; and
FIG. 5 depicts the metabolic activity of cells exposed to the nanosphres of FIG. 1 prepared in PBS.

### DETAILED DESCRIPTION OF THE PREFERRED EMBODIMENTS

The polymers of the present invention are A-B-A type triblock copolymers. The A end blocks are water-soluble, hydrophilic, and non-toxic polymer, preferrably selected from poly(alkylene oxides), and the B middle block oligomer is a polyarylate mid-block copolymerized from a tyrosine-derived diphenol and a diacid, linked together by an ester bond between the phenolic hydroxyl group of the tyrosine-derived diphenol and the carboxylic acid group of the diacid.

Among the more preferred poly(alkylene oxides) end blocks are polyethylene glycol, polypropylene glycol, polybutylene glycol, Pluronic polymers, and the like. Polyethylene glycols are preferred.

The polyarylate oligomer middle blocks of the present invention are prepared by condensation of a diacid with a diphenol according to the method described by U.S. Patent No. 5,216,115 in which diphenol compounds are reacted with aliphatic or aromatic dicarboxylic acids in a carbodiimide mediated direct polyesterification using 4-(dimethyl-amino)-pyridinium-p-toluene sulfonate (DPTS) as a catalyst. The disclosure of U.S. Patent No. 5,216,115 in this regard is incorporated herein by reference. Bis-diacids are selected as the polyarylate oligomer middle blocks to permit the A end blocks to be coupled at each end of the oligomer.

The diphenol compounds are the tyrosine-derived diphenol monomers of U.S. Patent Nos. 5,587,507 and 5,670,602, the disclosures of both of which are also incorporated herein by reference. The polyarylates are prepared using tyrosine-derived diphenol monomers having the structure of Formula III: 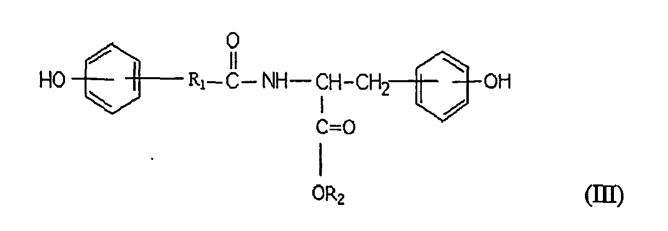 wherein R₁ and R₂ are the same as described above with respect to Formula I.

The preferred diphenol monomers are desaminotyrosyl-tyrosine carboxylic acids and esters thereof, wherein R₁ is -CH₂-CH₂-, which are referred to as DT esters. For purposes of the present invention, the ethyl ester (R₂ = ethyl) is referred to as DTE, the benzyl ester (R₂ = benzyl) as DTBn, and so forth. Both patents disclose methods by which these monomers may be prepared. For purposes of the present invention, the desaminotyrosyl-tyrosine free carboxylic acid (R₂ = hydrogen) is referred to as DT.

It is not possible to polymerize the polyarylate oligomers having pendant free carboxylic acid groups from corresponding diphenols with pendant free carboxylic acid groups without cross-reaction of the free carboxylic acid groups with the co-monomer. Accordingly, polyarylate oligomers that are homopolymers or copolymers of benzyl ester diphenyl monomers such as DTBn may be converted to corresponding free carboxylic acid homopolymers and copolymers through the selective removal of the benzyl groups by the palladium catalyzed hydrogenolysis method disclosed by co-pending and commonly owned U.S. Patent No. 6,120,491. The disclosure of this patent is incorporated by reference. The catalytic hydrogenolysis is necessary because the lability of the polymer backbone prevents the employment of harsher hydrolysis techniques.

Iodine- and bromine-containing polymers are radio-opaque. These polymers and their methods of preparation are disclosed by U.S. Patent No. 6,475,577. The disclosure of this patent is incorporated herein by reference. Radio-opaque polymers include repeating structural units in which one or more hydrogens of an aromatic ring, an alkylene carbon, or both, are replaced with an iodine or bromine atom. The triblock copolymers of the present invention may be similarly iodine- and bromine- substituted. Copolymers according to the present invention comprising the repeating structural units of Formula I are radio-opaque when copolymerized with radio-opaque monomers so that the copolymers also contain radio-opaque repeating structural units, preferably one or more of the A or B blocks in which one or more hydrogens of an aromatic ring, an alkylene carbon, or both, have been replaced with an iodine or bromine atom.

The polyarylate oligomer dicarboxylic acids have the structure of Formula IV:

HOOC-R-COOH (IV)

wherein R is the same as described above with respect to Formula I, and preferably contains up to 12 carbon atoms. R is preferably selected so that the dicarboxylic acids employed as starting materials are either important naturally-occurring metabolites or highly biocompatible compounds. Preferred Formula IV dicarboxylic acids therefore include the intermediate dicarboxylic acids of the cellular respiration pathway known as the Krebs cycle. These dicarboxylic acids include alpha-ketoglutaric acid, succinic acid, fumeric acid, malic acid and oxaloacetic acid, for which R is -CH₂-CH₂-C(=O)-, -CH₂-CH₂-, -CH=CH=, -CH₂-CH(-OH)- and -CH₂-C(=O)-, respectively.

Another naturally-occurring, preferred dicarboxylic acid is adipic acid (R₄ = (-CH₂-)₄), found in beet juice. Other preferred biocompatible dicarboxylic acids include oxalic acid (no R₄), malonic acid (R₄ = -CH₂-), glutaric acid (R₄ = (CH₂-)₃, pimellic acid (R₄ = (-CH₂-)₅, suberic acid (R₄ = (-CH₂-)₆ and azalaic acid (R₄ = (-CH₂-)₇. In other words, among the dicarboxylic acids suitable for use in the present invention are compounds in which R₄ represents (-CH₂-)_{z} wherein z is an integer between 0 and 12, inclusive. A preferred class of highly biocompatible aromatic dicarboxylic acids are the bis(p-carboxyphenoxy) alkanes such as bis(p-carboxyphenoxy) propane.

Preferred polyarylate oligomers have weight average molecular weights between about 1,000 and 50,000 daltons, preferably between about 3,000 and 25,000 daltons, and more preferably between about 5,000 and 15,000 daltons. Molecular weights are calculated by gel permeation chromatography relative to polystyrene standards in tetrahydrofuran without further correction. The triblock copolymers thus have weight average molecular weights between about 2,500 and 75,000 daltons, preferably between about 5,000 and 50,000 daltons, and more preferably between about 10,000 and 25,000 daltons.

The triblock copolymers are prepared by the reaction of a non-functionalized poly(alkylene oxide) mono-alkyl ether with an excess of either the dicarboxylic acid (mediated by a coupling agent such as dicyclohexyl carbodiimide). The following is a specific example of this general design, illustrating the synthesis of PEG-oligo-(DTO suberate)-PEG: 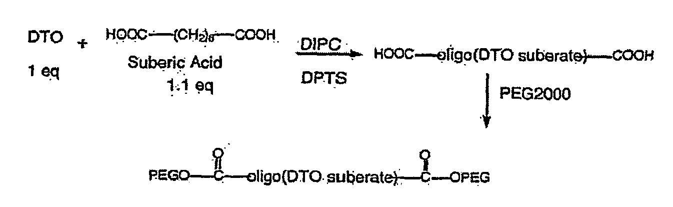 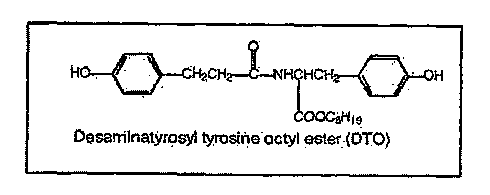 The molecular weights of the triblock copolymers can be controlled either by limiting the reaction time or the ratios of the components. Molecular weights can also be controlled by the quantity of the carbodiimide coupling reagent that is used.

The triblock copolymers degrade by hydrolysis into the original starting materials, i.e., the tyrosine-derived diphenols, the dicarboxylic acids, and the water-soluble, hydrophilic, and non-toxic polymer end blocks. The inventive copolymers are highly hydrophilic, which is advantageous for nanosphere drug delivery systems. However, the hydrophilic:hydrophobic balance of the copolymers can be varied in several ways. The ester of the pendant chain of the diphenol can be changed, with longer-chain ester groups increasing hydrophobicity. Increasing the molecular weight of the A end blocks, for example, by increasing the number of carbons in the alkylene group of a poly(alkylene oxide) will also increase hydrophobicity. Changing the dicarboxylic acid will also change the hydrophilic:hydrophobic balance.

The triblock copolymers of the present invention form vesicular structures in dilute aqueous solutions in the 5 - 200 nm range (diameter). Preferred structures have diameters between 50 and 150 nm. For example, poly(ethylene glycol)-block-oligo-(DTO suberate)-block-poly(ethylene glycol), i.e., PEG-oligo-(DTO suberate)-PEG triblock copolymer, forms vesicular structures in dilute aqueous solution having a diameter of about 100 nm range. The vesicles are characterized with conventional techniques, i.e., light scattering.

The triblock copolymers thus can be used to form into nanosphere drug and gene delivery systems. The synthesis of a triblock copolymer comprised of non-cytotoxic and biodegradable building blocks and capable of forming nanospheres (hollow vesicles) by a self-assembly process is important for use in many biomedical applications including but not limited to the use as a carrier for drugs or genetic materials. It is well established that the self-assembly of amphiphilic molecules depends on several correlated properties of the underlying material, i.e., its chemical structure, architecture or molecular weight. However, assuming that the driving force of the self-assembly is mainly governed by hydrophobic interactions, the design of a self-assembling block copolymer inherently depends on its molecular weight and hydrophobic to hydrophilic balance. The self-assembly of the triblock copolymers in dilute aqueous solution is induced using conventional injection combined with extrusion techniques. Active products are encapsulated by forming the nanospheres in solutions or suspensions of the product to be encapsulated.

The present invention therefore also includes injectable delivery systems for biologically and pharmaceutically active compounds formed by encapsulating the active compound with the polymer in a solution suitable for injection. The delivery system and its method of preparation are particularly well suited for use with active compounds such as pharmacologically active proteins, peptides, vaccines and genes, and the like, as well as with other small pharmacologically active molecules and contrast agents.

Nanospheres encapsulating an agent to be delivered may also be dispersed as a reservoir of the agent within the polymeric matrix of controlled release device. The host polymeric matrix may be a hydrogel or other bioerodible polymer. Such dispersions would have utility, for example, as active agent depots in transdermal drug delivery devices.

The delivery systems of the present invention are suitable for applications where localized delivery is desired, as well as in situations where systemic delivery is desired. Therapeutically effective dosages may be determined by either in vivo or in vitro methods. For each particular compound of the present invention, individual determinations may be made to determine the optimal dosage required. The range of therapeutically effective dosages will naturally be influenced by the route of administration, the therapeutic objectives, and the condition of the patient. For the various suitable routes of administration, the absorption efficiency must be individually determined for each active compound by methods well known in pharmacology. Accordingly, it may be necessary for the therapist to titer the dosage and modify the route of administration as required to obtain the optimal therapeutic effect. The determination of effective dosage levels, that is, the dosage levels necessary to achieve the desired result, will be within the ambit of one skilled in the art. Typically, applications of compound are commenced at lower dosage levels, with dosage levels being increased until the desired effect is achieved. The release rate of the active compound from the formulations of this invention are also varied within the routine skill in the art to determine an advantageous profile, depending on the therapeutic conditions to be treated.

A typical dosage might range from about 0.001 mg/kg to about 1000mg/kg, preferably from about 0.01 mg/kg to about 100 mg/kg, and more preferably from about 0.10 mg/kg to about 20 mg/kg. Advantageously, the compounds of this invention may be administered several times daily, and other dosage regimens may also be useful.

The compositions may be administered subcutaneously, intramuscularly, colonically, rectally, nasally, orally or intraperitoneally, employing a variety of dosage forms such as suppositories, implanted pellets or small cylinders, aerosols, oral dosage formulations and topical formulations, such as ointments, drops and transdermal patches.

Acceptable pharmaceutical carriers for therapeutic use are well known in the pharmaceutical field, and are described, for example, in *Remington's Pharmaceutical Science,* Mac Publishing Co., (A.R. Gennaro edt. 1985). Such materials are non-toxic to recipients at the dosages and concentrations employed, and include diluents, solubilizers, lubricants, suspending agents, encapsulating materials, solvents, thickeners, dispersants, buffers such as phosphate, citrate, acetate and other organic acid salts, anti-oxidants such as ascorbic acid, preservatives, low molecular weight (less than about 10 residues) peptides such as polyarginine, proteins such as serum albumin, gelatin or immunoglobulins, hydrophilic polymers such as poly(vinylpyrrolindinone), amino acids such as glycine, glutamic acid, aspartic acid or arginine, monosaccharides, disaccharides, and other carbohydrates including cellulose or its derivatives, glucose, mannose or dextrines, chelating agents such as EDTA, sugar alcohols such as mannitol or sorbitol, counter-ions such as sodium and/or non-ionic surfactants such as tween, pluronics or PEG.

The polymer-drug combinations of this invention may be prepared for storage under conditions suitable for the preservation of drug activity as well as maintaining the integrity of the polymers, and are typically suitable for storage at ambient or refrigerated temperatures. Sterility may be readily accomplished by conventional methods.

Gene transfection is performed by contacting cells to be transfected with buffered suspensions or solutions of plasmid vectors encapsulated by the polymers of the present invention. The means by which plasmid vectors are prepared and gene transfection is otherwise accomplished are well known to those skilled in the art and do not require description.

The following non-limiting examples set forth hereinbelow illustrate certain aspects of the invention. All parts and percentages are by weight unless otherwise noted and all temperatures are in degrees Celsius. Dicarboxylic acids and all other reagents were purchased in pure form and were used as received. Solvents were of "HPLC grade." Diphenolic monomers (e.g., the esters of desamino tyrosil-tyrosine) were prepared according to the procedure provided in Example I of U.S. Patent No. 5,099,060. Although this procedure refers specifically to DTH, monomers having esters other than the hexyl ester can be readily prepared by the same basic procedure. The DPTS catalyst was prepared as described by Moore, *et al.,* Macromol., 23(1), 65-70 (1990).

### EXAMPLES

### Example 1: Preparation of poly(ethylene glycol)-block-oligo-(DTO suberate)-block-poly(ethylene glycol) self-assembling nanospheres, abbreviated as PEG-oligo-(DTO-suberate)-PEG) triblock copolymer.

**Chemicals:** Desaminotyrosyl tyrosine octyl ester (DTO) was prepared using known procedures. Methylene chloride (HPLC grade), 2-propanol and methanol, were obtained from Fisher Scientific, Pittsburgh, PA and used without purification. Suberic acid, 4-dimethylaminopyridine, 4-toluenesulfonic acid, and poly(ethylene glycol) monomethyl ether (Mw of 2000 gmol-1) were obtained from Aldrich Chemical Co, Milwaukee, WI, and used without purification. Diisopropylcarbodiimide was obtained from Tanabe Chemicals.

**Synthesis procedure:** In a 100 mL round-bottomed flask were placed 2.21 g (0.005 mol) of desamino-tyrosyl tyrosine octyl ester (DTO), 0.96 g (0.0055 mol) of suberic acid, 0.59 g (0.002 mol) of 4-dimethylaminopyridinium-p-toluene sulfate and 25 mL of methylene chloride and stirred at 293k. To the stirred suspension was added 1.8 g (0.014 mol) of diisopropylcarbodiimide and stirring was continued. After 1 h an aliquot was withdrawn and analyzed by GPC which showed a Mₙ of 7.03 kgmol⁻¹ and M_{w} of 14.9 kgmol⁻¹ (polystyrene equivalent). To the reaction mixture was then added 1.1 g of poly(ethylene glycol) monomethyl ether (M_{w} of 2000 kgmol⁻¹), and 0.4 g of diisopropylcarbodiimide. After 2 h, the reaction mixture was filtered using a sintered glass funnel and the filtrate was concentrated to a volume of 10 mL and then precipitated with 2-propanol. The precipitate was dried, redissolved in 10 mL of methylene chloride and precipitated with 50 mL of methanol. The precipitate was isolated by centrifugation, washed with 20 mL of methanol and then dried under vacuum at room temperature. The product was characterized by GPC (M_{w} and Mₙ), ¹H NMR (CDC13, 400 MHz): 6.98-7.20 ppm (Ar-H), 5.98 (d, NH), 4.86 (d, CH of tyrosine), 4.08 (m, OCH2 of DTO), 3.65 (CH2CH2 of PEG), 3.38 (s, OCH3 of PEG), and elemental analysis.

**Preparation of vesicles:** The self-assembly of the polymer was induced using conventional injection combined to extrusion techniques. 10 mg of the polymer was dissolved in 0.2 mL of THF in a scintillation vial. This solution was added dropwise to 5 mL of Nanopure water under mild agitation. The resulting turbid dispersion was sequentially filtered through 0.45, 0.22 and 0.1 micrometer nylon syringe filters. The filtrate from the final extrusion was used for all the subsequent characterizations.

**Freeze-fracture Transmission Electron Microscopy :** A drop of the vesicle dispersion was rapidly frozen in liquid propane chilled to 123K with liquid nitrogen. The sample was then freeze-fractured to shape Platinum/Carbon replica (High Vacuum Freeze-Etch Unit, Balzers Union Limited, FL-9496, Principality of Lichtenstein). The resulting replicas were subsequently collected on 200-mesh copper grids to be analyzed by transmission electron microscopy in a JEM-100CXII electron microscope operating at 80 kV (JEOL LTD., Tokyo, Japan).

**Dynamic light scattering:** A submicron particle sizer (PSS Nicomp, Particle sizing systems, Santa Barbara, California, USA) calculates the photon intensity autocorrelation function g²(t). The samples were prepared by filtering the solutions through 0.45 m Millipore membranes into 6*50 mm borosilicate cells. The experiments were performed at 303K. The data of DLS were treated by a Cumulant analysis.

**Cell Cytotoxicity Assay:** URM-106 cells were maintained and treated with different concentrations of vesicles (as specified below). After 4 hours in the incubator, the cytotoxicity of vesicles was determined by MTS assay. This assay is composed of solutions of a novel tetrazolium compound (3-(4,5-dimethylthiazol-2-yl)-5-(3-carboxymethoxyphenyl)-2-(4-sulfophenyl)-2H-tetrazolium, inner salt; MTS and an electron coupling reagent (phenazine methosulfate) PMS. MTS is bioreduced by cells into a formazan that is soluble in tissue culture medium.. Briefly, the cells were seeded into 24-well plates at a density of 50,000 cells per well. After 24 hours, the culture medium was replaced with 400 mL of DMEM containing various concentrations of polymeric vesicles (2 mg/mL, 1 mg/mL, 0.5 mg/mL, 0.4 mg/mL, 0.2 mg/mL, 0.1 mg/mL) and the cells were incubated for 4 hours at 37°C. Thereafter, Cell Titer 96 Aqueous One Solution Reagent (Promega, Madison, WI) was added to each well and incubated for another 2 hours. 80 L of that solution was transferred to a 96-well microplate and absorbance was determined using an absorbance plate reader (PowerWave X, Bio-Tek Instruments, Inc., Highland Park, VE) at 490 nm.

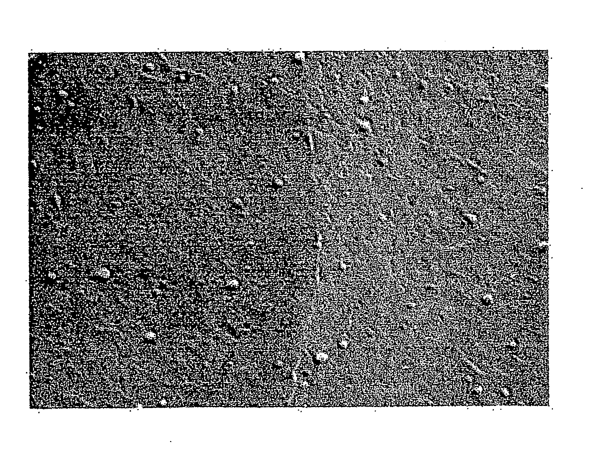

Figure 1 is a transmission electron micrograph obtained after freeze fracturing of the resulting self-assembly. From the electron micrograph, the PEG-oligo-(DTO suberate)-PEG triblock copolymer self-assembly appears to lead to spherical structures of 100 nm size. The size distribution appears to be quite large. Nonetheless, the sample preparation condition of freeze fracturing being somehow destructive, some of the particles might have been partially destroyed or buried in the replica matrix during the freeze fracturing process.

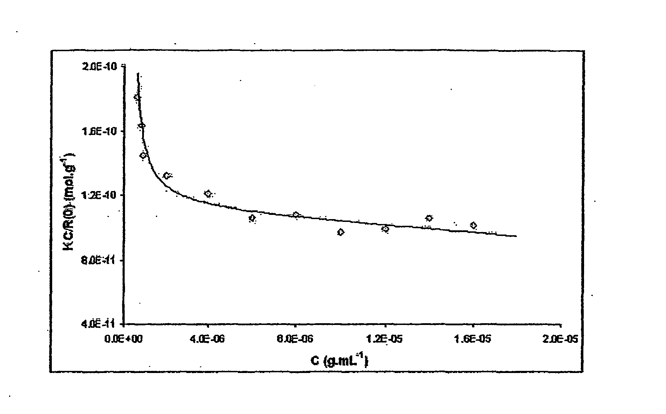

In order to get a proper and reliable estimation of the size of those spherical particles, static light scattering investigations have been performed. Figure 2 represents a typical Zimm diagram on which, for clarity, only the extrapolated values to zero scattering angle of the inverse scattered intensity have been represented. From this concentration profile, one can observe two regimes, below and above a 4 microgram per mL polymer concentration. The decrease of the scattered intensity at low concentrations is the fingerprint of a critical aggregation concentration, CAC. This is not surprising as the self-assembly of amphiphilic molecules is a reversible process that leads for instance to the disintegration of the nanostructures upon dilution. Assuming a close association model, above 4 microgram per mL, lonely spherical structures are swimming in the solution. Below this concentration, the self-assembled particles start disintegrating into individual PEG-oligo-(DTO suberate)-PEG triblock copolymer molecules. Therefore the two objects, the nanoparticles and the individual particles-building macromolecules coexist in the aqueous solution.

From these results, the critical aggregation concentration (CAC) could be determined to be about 0.26 4 microgram per mL. Unexpectedly, this value is rather low (three orders of magnitude) when compared with previously described self-assembling block copolymer systems. This low CAC confers to this system an unexpected stability upon dilution which is a major technological advantage over previously described systems of this kind.

Full characterization of the spherical structures is obtained from the linear regime of the concentration profile of the inverse scattered intensity. The results are presented in Table 1. As expected, these aggregates have a radius of gyration of 51 nm, which is in good agreement with the largest spherical structures observed in the freeze fractured samples analyzed by transmission electron microscopy (Figure 1).

**TABLE 1**

| LS study | |
|---|---|
| dn/dc (mL.g⁻¹) | 1.23 |
| cac (µg.mL⁻¹) | 0.26 |
| M(10⁹ g.mol⁻¹) | 9 |
| A₂(10⁻⁷mol.mL.g²). | -5 |
| R_{g}(nm) | 51 |
| Rₕ(nm) | 49 |
| D₀ (10⁻⁸ cm².s⁻¹) | 5.6 |
| p (10⁵) | 6.38 |
| p | 1.04 |

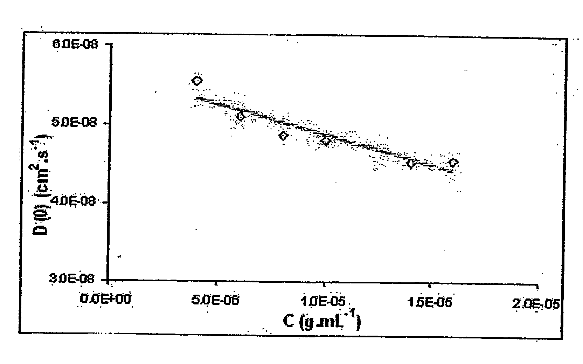

Besides their large radius, those investigations do not allow to claim that these spheres are hollow. Therefore, in a following set of experiments, dynamic light scattering investigations were carried out. Figure 3 shows the dynamic Zimm plot obtained after extrapolation of the diffusion coefficient to zero scattering angle. The linearity of the angular profile of the diffusion coefficient (data not shown) ensures that a single diffusive process occurs in this system. The monodispersity of the dispersion is supported by a reasonable polydispersity index of 1.3. Using the Stokes-Einstein relation, the hydrodynamic radius of the nanoparticles is found to be the same than their radius of gyration (within the accuracy limit of the light scattering techniques). As foreseen, PEG-oligo-(DTO suberate)-PEG triblock copolymer self assemble into 100 nm hollow sphere structures, i.e , vesicles, a size suitable for gene delivery to cells to be transfected.

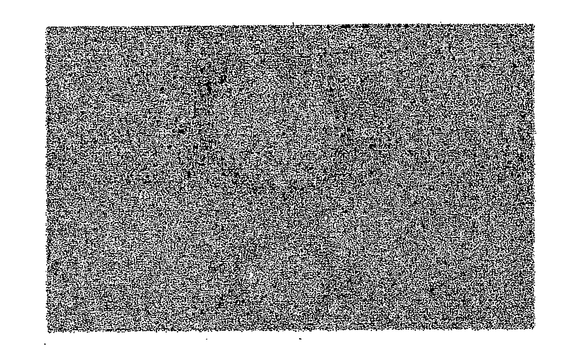

This hollow sphere morphology was further confirmed by transmission electron microscopy, showing clearly hollow spheres with an outer membrane having a thickness of about 6 nanometer, shown in Figure 4.

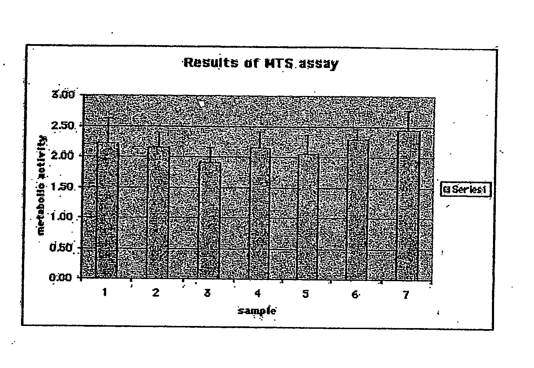

As previously mentioned, the building blocks of the amphiphilic block copolymer are known to be non-cytotoxic. Cytotoxicity assay was performed using the PEG-oligo-(DTO suberate)-PEG triblock copolymer vesicles prepared in PBS. As the polymer does not carry charges, no change upon the vesicle size was detected by light scattering (data not shown). In order to eliminate any organic solvent traces, the vesicles were purified by size exclusion chromatography, SEC. As it will be further used for encapsulation purposes this technique has been chosen over a more straightforward organic solvent blow-drying or dialysis. The results of this assay performed with different polymer concentrations done in serum-free medium are shown on Fig. 5. In the investigated concentration range (2 mg mL⁻¹ to 0.1 mg mL⁻¹), no significant decrease of the cell metabolic activity was detected, confirming that the PEG-oligo-(DTO suberate)-PEG vesicles do not induce any cytotoxicity. Therefore it was concluded that these polymeric vesicles are non-toxic and well tolerated by cells, at least in short-time exposures.

### Example 2: Gene Delivery

**DNA encapsulation:** The PEG-oligo-(DTO suberate)-PEG triblock copolymer of Example 1 was dissolved in THF at a concentration of 5 mg/mL in THF. 30 g of DNA was suspended in 4.79 mL of PBS. The polymer solution was then added dropwise to the DNA suspension under constant stirring. Vesicles were purified of any residual THF and unencapsulated DNA by column chromatography using Sepharose HR 400 (Biorad, Piscataway, NJ). The resulting vesicle suspension was of 0.5 mg/mL.

**Transfections:** All transfections were performed using pEGFP-actin (Clonetech) plasmid. Control transfections were carried out using the Superfect reagent (Qiagen, Valencia CA) using 1 g of DNA with 4 1 of the Superfect reagent according to manufacturer's specification. UMR-106 cells were seeded the day prior to transfection in 24-well plates at a density between 40-60% confluence per well. On the day of transfection cells were rinsed once with PBS and the following transfection conditions were set up:
1. Cells exposed to 0.5 mg/mL vesicle suspension in PBS.
2. Cells exposed to 0.25 mg/mL vesicle suspension diluted in serum-free DMEM or 20% serum-containing DMEM
3. Cells exposed to 0.1mg/mL vesicle suspension diluted in serum-free DMEM or 20% serum containing DMEM.
4. Cells exposed to 0.05 mg/mL vesicle suspension diluted in serum-free DMEM or 20% serum-containing DMEM.

Transfections were carried out for 4 hours at which point cells were washed once with PBS and transfection medium was replaced with DMEM medium supplemented with 10% FBS, 100 units/mL penicillin, 100 g/mL streptomycin. Cells were incubated for approximately 48 hours at which point the transfection efficiency was assessed both by fluorescent confocal microscopy and flow cytometry.

This was a preliminary experiment, and therefore the transfection efficiency was only about 1-2% of all cells. However, it is obvious that upon slight modification of the transfection protocol, significantly higher transfection rates will be obtained. It is particularly important to note that there seems to be very little or no difference in the transfection efficiency between the experiments carried in the presence or absence of fetal bovine serum. This is probably due to the protein repellant property of the PEG end blocks of the polymer. This finding is especially important for *in vivo* applications where particles become instantaneously deactivated due to opsonization (adsorption of serum proteins).

The present application claims priority benefit of U.S. Provisional Patent Application No. 60/378,042 filed May 15, 2002, the disclosure of which is incorporated by reference.

The foregoing examples and description of the preferred embodiments should be taken as illustrating, rather than as limiting the present invention as defined by the claims. As will be readily appreciated, numerous variations and combinations of the features set forth above can be utilized without departing from the present invention as set forth in the claims. Such variations are not regarded as a departure from the spirit and script of the invention, and all such variations are intended to be included within the scope of the following claims.

## Claims

1. A triblock copolymer having an A-B-A structure wherein each A end block is a water-soluble, hydrophilic, and non-toxic polymer end block; and the B middle block is an polyarylate oligomer with the same or different repeating units having the structure: 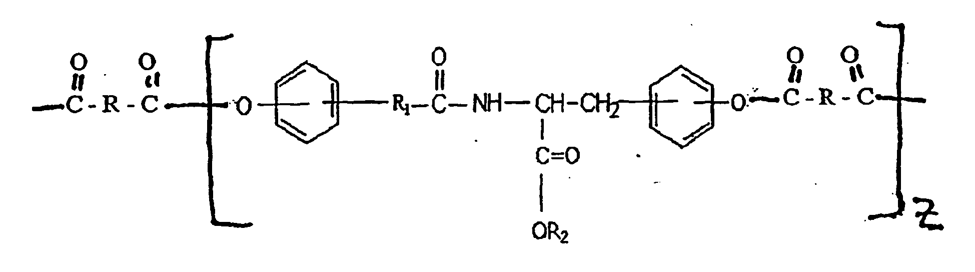 wherein Z is between 2 and to about 20, R₁ is CH=CH or(CH₂)ₙ wherein n is from 0 to 18, inclusive; R₂ is selected from the group consisting of hydrogen and straight and branched alkyl and alkylaryl groups containing up to 18 carbon atoms; and R is selected from the group consisting of a bond or straight and branched alkyl and alkylaryl groups containing up to 18 carbon atoms.

2. The triblock copolymer of claim 1, wherein said end blocks are poly (alkylene oxides) having the structure:
R₃- [ (CH₂-) ₐCHR₃-0-]m-
wherein m for each A is independently selected to provide a molecular weight for each A between about 1000 and about 15,000 and R₃ for each A and within each A is independently selected from the group consisting of hydrogen and lower alkyl groups containing from one to four carbon atoms.

3. The triblock copolymer of claim 1, wherein said end blocks have the structure CH₃O- [CH₂-CH₂-O-]ₘ.

4. The triblock copolymer of claim 1, wherein Z is about 10.

5. The triblock copolymer of claim 1, wherein one or more of R, R₁ and R₂ contains an ether linkage.

6. The triblock copolymer of claim 1, wherein R₁ is-CH₂-CH₂-.

7. The triblock copolymer of claim 1, wherein R₂ is selected from the group consisting of ethyl, butyl, hexyl, octyl and benzyl groups.

8. The triblock copolymer of claim 1, wherein R contains up to 12 carbon atoms.

9. The polyarylate of claim 8, wherein R is selected from the group consisting of-CH₂-CH₂-C (=O)-,-CH=CH-,-CH₂-CH (-OH)-,-CH₂-C (=O)- and (-CH₂-)_{z}, wherein z is between 0 and 12, inclusive.

10. Nanospheres formed from the triblock copolymer of claim 1.

11. A nanosphere-encapsulated active compound, for administering to a patient in need thereof, wherein the encapsulating nanospheres are formed from the triblock copolymer of claim 1.

12. The nanosphere-encapsulated compound of claim 11, wherein the encapsulated compound is a contrast agent.

13. The nanosphere-encapsulated compound of claim 11, wherein the encapsulated compound is a biologically or pharmaceutically active compound.

14. A composition for delivering an agent to a patient in need thereof comprising a pharmaceutically acceptable carrier and an effective amount of nanospheres encapsulating said agent with the triblock copolymer of claim 1.

15. The composition of claim 14, wherein said agent is a contrast agent.

16. The composition of claim 14, comprising a pharmaceutical composition comprising a nanosphere-encapsulated biologically or pharmaceutically active compound, wherein said active compound nanospheres are present in an amount sufficient for therapeutically effective site-specific or systemic delivery.

17. The pharmaceutical composition of claim 16, wherein said nanospheres are embedded or dispersed in a drug delivery polymer matrix.

18. The pharmaceutical composition of claim 16, wherein said active compound is a pharmacologically active protein, peptide, vaccine or gene.

## Patentansprüche

1. Triblock-Copolymer mit einer A-B-A-Struktur, in der jeder A-Endblock ein wasserlöslicher, hydrophiler und nicht-toxischer Polymer-Endblock ist; und der B-Mittelblock ein Polyarylat-Oligomer mit den gleichen oder verschiedenen Struktureinheiten ist, welche die Struktur aufweisen: 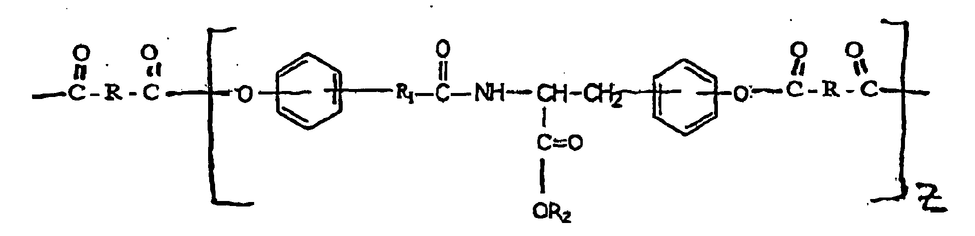 in der Z zwischen 2 und etwa 20 ist, R₁ für CH=CH oder (CH₂)ₙ steht, worin n O bis 18 einschließlich ist; R₂ ausgewählt ist aus der Gruppe bestehend aus Wasserstoff und geraden und verzweigten Alkyl- und Alkylarylgruppen, die bis zu 18 Kohlenstoffatome enthalten; und R ausgewählt ist aus der Gruppe bestehend aus einer Bindung oder geraden oder verzweigten Alkyl- oder Alkylarylgruppen, die bis zu 18 Kohlenstoffatome enthalten.

2. Triblock-Copolymer nach Anspruch 1, in dem die Endblöcke Poly(alkylenoxide) mit der Struktur
R₃-[(CH₂-)ₐCHR₃-O-]ₘ-
sind, worin m bei jedem A unabhängig so ausgewählt ist, dass ein Molekulargewicht von jedem A zwischen etwa 1000 und etwa 15000 geliefert wird, und R₃ bei jedem A und innerhalb jedes A unabhängig ausgewählt ist aus der Gruppe bestehend aus Wasserstoff und Niederalkylgruppen, die 1 bis 4 Kohlenstoffatome enthalten.

3. Triblock-Copolymer nach Anspruch 1, in dem die Endblöcke die Struktur CH₃O-[CH₂-CH₂-O-]ₘ aufweisen.

4. Triblock-Copolymer nach Anspruch 1, in dem Z für etwa 10 steht.

5. Triblock-Copolymer nach Anspruch 1, in dem eines oder mehrere von R, R₁ und R₂ eine Ether-Verknüpfung enthalten.

6. Triblock-Copolymer nach Anspruch 1, in dem R₁ für -CH₂-CH₂- steht.

7. Triblock-Copolymer nach Anspruch 1, in dem R₂ ausgewählt ist aus der Gruppe bestehend aus einer Ethyl-, Butyl-, Hexyl-, Octyl- und Benzylgruppe.

8. Triblock-Copolymer nach Anspruch 1, in dem R bis zu 12 Kohlenstoffatome enthält.

9. Polyarylat nach Anspruch 8, in dem R ausgewählt ist aus der Gruppe bestehend aus -CH₂-CH₂-C(=O)-, -CH=CH-, -CH₂-CH(-OH)-, -CH₂₋C(=O)- und (-CH₂-)_{z}, worin z zwischen 0 und 12 einschließlich ist.

10. Nanokügelchen, gebildet aus dem Triblock-Copolymer nach Anspruch 1.

11. In Nanokügelchen eingekapselte aktive Verbindung zur Verabreichung an einen Patienten, der sie benötigt, wobei die einkapselnden Nanokügelchen aus dem Triblock-Copolymer nach Anspruch 1 gebildet sind.

12. In Nanokügelchen eingekapselte Verbindung nach Anspruch 11, wobei die eingekapselte Verbindung ein Kontrastmittel ist.

13. In Nanokügelchen eingekapselte Verbindung nach Anspruch 11, wobei die eingekapselte Verbindung eine biologisch oder pharmazeutisch aktive Verbindung ist.

14. Zusammensetzung zur Zufuhr eines Mittels an einen Patienten, der es benötigt, umfassend einen pharmazeutisch annehmbaren Träger und eine wirksame Menge von Nanokügelchen, die das Mittel in dem Triblock-Copolymer nach Anspruch 1 einkapseln.

15. Zusammensetzung nach Anspruch 14, in der das Mittel ein Kontrastmittel ist.

16. Zusammensetzung nach Anspruch 14, umfassend eine pharmazeutische Zusammensetzung, die eine in Nanokügelchen eingekapselte biologisch oder pharmazeutisch aktive Verbindung umfasst, wobei die Nanokügelchen mit aktiver Verbindung in einer Menge vorliegen, die für eine therapeutisch wirksame ortsspezifische oder systemische Zufuhr ausreicht.

17. Pharmazeutische Zusammensetzung nach Anspruch 16, in der die Nanokügelchen in einer Arzneistoffzufuhr-Polymermatrix eingebettet oder dispergiert sind.

18. Pharmazeutische Zusammensetzung nach Anspruch 16, in der die aktive Verbindung ein pharmakologisch aktives bzw. aktiver Protein, Peptid, Impfstoff oder Gen ist.

## Revendications

1. Copolymère tribloc ayant une structure A-B-A dans laquelle chaque bloc d'extrémité A est soluble dans l'eau, hydrophile, et de bloc d'extrémité polymère non toxique ; et le bloc intermédiaire B est un oligomère polyarylate avec des unités répétitives différentes ayant la structure : 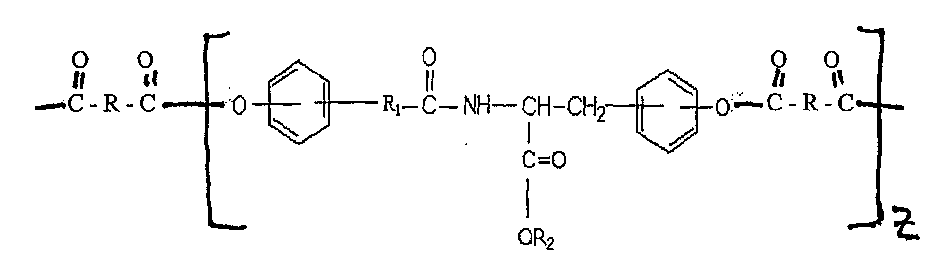 dans laquelle Z a une valeur entre 2 et 20 environ, R₁ est CH=CH ou (CH₂)ₙ dans laquelle n a une valeur de 0 à 18, compris ; R₂ est choisi parmi le groupe composé de l'hydrogène des groupes alkyle et alkylaryle droits ou ramifiés contenant jusqu'à 18 atomes de carbone ; et R est choisi parmi le groupe comprenant une liaison ou des groupes alkyle et alkylaryle droits ou ramifiés contenant jusqu'à 18 atomes de carbone.

2. Copolymère tribloc selon la revendication 1, dans lequel lesdits blocs d'extrémité sont des polyoxydes d'alkylène ayant la structure :
R₃- [ (CH₂-) ₐCHR₃-O-]ₘ-
dans laquelle m pour chaque A est indépendamment choisi pour fournir un poids moléculaire pour chaque A entre 1000 environ et 15000 environ et R₃ pour chaque A et dans chaque A est choisi de façon indépendante parmi le groupe composé de l'hydrogène et des groupes alkyle inférieurs contenant de un à quatre atomes de carbone.

3. Copolymère tribloc selon la revendication 1, dans lequel lesdits blocs d'extrémité ont la structure CH₃O-[CH₂₋CH₂-O-]ₘ.

4. Copolymère tribloc selon la revendication 1, dans lequel Z a la valeur 10 environ.

5. Copolymère tribloc selon la revendication 1, dans lequel un ou plusieurs de R, R₁ et R₂ contiennent une liaison éther.

6. Copolymère tribloc selon la revendication 1, dans lequel R₁ est -CH₂-CH₂-.

7. Copolymère tribloc selon la revendication 1, dans lequel R₂ est choisi parmi le groupe composé des groupes éthyle, butyle, hexyle, octyle et benzyle.

8. Copolymère tribloc selon la revendication 1, dans lequel R contient jusqu'à 12 atomes de carbone.

9. Polyarylate selon la revendication 8, dans lequel R est choisi parmi le groupe composé de -CH₂-CH₂-C(=O)-, -CH=CH-, -CH₂-CH (-OH) -, -CH₂-C(=O)- et (-CH₂-)_{z}, dans laquelle z a une valeur entre 0 et 12, compris.

10. Nanosphères formées à partir du copolymère tribloc de la revendication 1.

11. Composé actif encapsulé dans une nanosphère, pour l'administration à un patient en ayant besoin, dans lequel les nanosphères d'encapsulation sont formées à partir du copolymère tribloc de la revendication 1.

12. Composé encapsulé dans une nanosphère selon la revendication 11, dans lequel le composé encapsulé est un agent de contraste.

13. Composé encapsulé dans une nanosphère selon la revendication 11, dans lequel le composé encapsulé est un composé actif sur le plan biologique ou pharmaceutique.

14. Composition pour la délivrance d'un agent à un patient qui en a besoin comprenant un support acceptable sur le plan pharmaceutique et une quantité efficace des nanosphères encapsulant ledit agent avec le copolymère tribloc de la revendication 1.

15. Composition selon la revendication 14, dans lequel ledit agent est un agent de contraste.

16. Composition selon la revendication 14, comprenant une composition pharmaceutique comprenant un composé actif sur le plan biologique ou pharmaceutique, dans lequel lesdites nanosphères de composé actif sont présentes dans une quantité suffisante pour une délivrance efficace sur le plan thérapeutique sur un site spécifique ou systémique.

17. Composition pharmaceutique selon la revendication 16, dans laquelle lesdites nanosphères sont incluses ou dispersées dans une matrice polymère de délivrance de médicament.

18. Composition pharmaceutique selon la revendication 16, dans laquelle ledit composé actif est une protéine, un peptide, un vaccin ou un gène actifs sur le plan pharmaceutique.
